# EUROPEAN PATENT SPECIFICATION

(11) **EP 4 217 027 B1**
(45) Date of publication and mention of the grant of the patent: **08.05.2024**
(21) Application number: 21782746.8
(22) Date of filing: 27.09.2021
(51) Int. Cl.: A61M 11/02, A61M 15/00, A61M 16/00, A61M 16/08, A61M 11/06

(54) **INHALATION DEVICE**
INHALIERVORRICHTUNG
DISPOSITIF D'INHALATION

(30) Priority: 28.09.2020 EP 20198619
(43) Date of publication of application: 02.08.2023
(73) Proprietor: Vectura Delivery Devices Limited, Chippenham, Wiltshire SN14 6FH (GB)
(72) Inventor: KOLB, Tobias, Cambridge Cambridgeshire CB4 0GZ (GB); MUELLINGER, Bernhard, Cambridge Cambridgeshire CB4 0GZ (GB); HOFFMANN, Tobias, Cambridge Cambridgeshire CB4 0GZ (GB); SWANBURY, Philip, Cambridge Cambridgeshire CB4 0GZ (GB); FREY, Manuel, Cambridge Cambridgeshire CB4 0GZ (GB); TAYLOR, Donal, Cambridge Cambridgeshire CB4 0DW (GB); HODGES, Jake, Cambridge Cambridgeshire CB4 0DW (GB); KARAVENGLEMAN, Mary, Cambridge Cambridgeshire CB4 0DW (GB); KANTOR, Erica, Cambridge Cambridgeshire CB4 0DW (GB)
(74) Representative: Clarke, Christopher John
(86) International application number: PCT/EP2021/076511
(87) International publication number: WO 2022/064041

(56) References cited:
- EP-A1- 2 316 514
- WO-A1-2013/160129
- WO-A1-2020/003305
- US-B1- 10 286 163

## Description

### Technical Field of the Invention

The present invention relates to an inhalation device for providing an aerosol flow and / or an air flow.

### Background

The therapeutic inhalation of aerosols is an effective and gentle method for the treatment of various respiratory diseases such as acute respiratory disease, chronic obstructive pulmonary disease, and bronchial asthma. The drugs (i.e. active pharmaceutical ingredients, known as APIs), are provided as an aerosol of droplets having the correct size to penetrate the patient's bronchi or lungs, where they are effective for the treatment of the disease. One type of device that is commonly used is a jet nebulizer. Jet nebulizers typically have a compressor which provides a supply of gas at sufficiently high pressure to disperse a liquid formulation (i.e. a solution or suspension of the drug) into inhalable droplets in a nebulizing nozzle. Some jet nebulizers are breath-actuated, so that aerosol generation only takes place during inhalation, in order to avoid wasting the drug.

EP 2 316 514 discloses a composite lung therapy device with a single source of pressurized gas and a Venturi to increase the flow volume. The device provides aerosol delivery combined with either pulsatile positive gas flow internal to the patient's airways to provide a secretion clearance mode, or with a linear positive gas flow. WO 2020/003305 discloses an aerosol generating device having a gas pump which delivers compressed gas to a porous medium that contains liquid. WO 2013/160129 discloses a system for delivering medicament having a pump that creates a flow of pressurized gas that breaks liquid medicament into a plurality of particles causing the atomized medicament to be delivered into the patient's lungs. US 10 286 163 discloses a hand-held aerosolizer that uses an air pump to deliver an aerosolized solution to the user when he or she inhales. US 8 181 644 discloses a breath-actuated jet nebulizer that actively provides a defined volume of air at a defined flow rate to the patient on each inhalation. The device inputs aerosolized drug particles into the inhalation flow at a specified time during each inhalation (known as an "aerosol bolus"). This allows the drug to be targeted on a particular region of the patient's lungs. For example, providing a period of drug-free air after the aerosolized drug ensures that the drug is delivered to the periphery of the lung; alternatively, the aerosolized drug can be provided close to the end of the inhalation, so that it passes through the upper airway and is delivered to the bronchial regions of the lung. The device has a compressor which generates a supply of pressurized air. In order to be able to switch the drug-containing air flow on and off whilst maintaining a constant overall flow rate, the pressurized air is split into two separate flows: an 'aerosol flow' through the nebulizing nozzle to generate aerosol droplets; and a 'support flow' to the mouthpiece at which the patient inhales, without going through the nozzle of the jet nebulizer. The aerosol flow is mixed with the support flow in a mixing means and the combined flow is then inhaled through a mouthpiece. In the aerosol-free part of the inhalation, the aerosol flow is switched by valves so that it bypasses the jet nebulizer and is provided directly to the mouthpiece together with the support flow. In this manner, a constant total flow rate to the patient is maintained regardless of whether the drug is being nebulized or not. However, switching the aerosol flow necessitates a system of valves. Moreover, a Venturi nozzle is needed in the support flow path to increase the air flow, otherwise a large compressor would be required. The Venturi also helps to smooth the inherent pressure variations in the output from the compressor. It would be desirable to provide a jet nebulizer which is capable of delivering an aerosol bolus, but without the complexity of the switching valves and the Venturi nozzle.

### Brief Description of the Invention

The present invention addresses this problem and, in a first aspect, provides a device for providing a flow of aerosol and / or air, comprising
- a base unit containing a first pump for providing a nebulizer air flow, a first channel that connects the first pump to a first air outlet, a second pump for providing a support air flow which is a multiple head diaphragm pump, such as a rolling diaphragm pump, or a rotary vane pump, or a scroll pump, a second channel that connects the second pump to a second air outlet, a pressure sensor connected to the second channel, and a controller for controlling the first and second pumps;
- a handset containing a reservoir for liquid to be nebulized, a mouthpiece and a mixing chamber, the mixing chamber having a first air inlet connected to a nebulizing nozzle for generating an aerosol flow from the nebulizer air flow and the liquid, and a second air inlet;
- a first tube connecting the first air outlet of the base unit to the first air inlet of the mixing chamber; and a second tube connecting the second air outlet of the base unit to the second air inlet of the mixing chamber,
so that the the aerosol flow and the support air flow are combined in the mixing chamber to provide a total flow through the mouthpiece to a patient.

The device provides an aerosol flow and a support flow, which can be independently varied by controlling the output flow rate from the first and second pumps. The device provides the same functionality as the device of US 8 181 644, in particular a defined volume of air and aerosol as a bolus at a defined flow rate on each inhalation, but without requiring the valves and Venturi nozzle. Moreover, having a separate support flow pump means that different flow rates can be selected by controlling the support flow pump. This allows the device to be adjusted to provide flow rates adapted to achieve a high deposition in the lung or peripheral airways and low deposition in the throat, for specific patient populations. Thus, unlike conventional nebulizers, a single device can be used for patient groups with different airway capacities, for example due to their age or lung function.

The first and second pumps may be different types of pump and so they may be chosen according to their particular function. The first pump may be a piston-type compressor. The second pump is a multiple head diaphragm pump, such as a rolling diaphragm pump, or a rotary vane pump, or a scroll pump; these types of pump provide a suitably high flow rate with a smooth flow. In particular, the first pump may be a piston-type compressor and the second pump may be a rolling diaphragm pump.

The device may further comprise a display screen for providing instructions and / or feedback to the patient or to a care-giver.

The first and / or second tubes may be from 1000 mm to 1300 mm in length and may have an inner diameter of from 3 to 5 mm.

The device may be configured to independently control the aerosol flow rate and / or the support air flow rate, for example to vary as a function of time.

The device may be configured so that each inhalation may have an aerosol period in which there is both support flow and aerosol flow so that aerosol is delivered to the patient, and may also have one or more aerosol-free periods when there is support flow but no aerosol flow, so that only air is delivered to the patient.

The device may be configured so that each inhalation may have an aerosol period followed by an aerosol-free period; or an aerosol-free period followed by an aerosol period; or a first aerosol-free period, followed by an aerosol period, followed by a second aerosol-free period.

The device may be configured so that the aerosol flow rate may be approximately constant during the aerosol period. The device may be configured so that the support flow rate may be higher during the aerosol-free period(s) so that the total flow rate is approximately constant throughout the aerosol and aerosol-free periods of the inhalation.

The device may be configured so that the support flow rate may be varied so that the total flow rate mimics the patient's natural breathing pattern. The support flow rate may also be varied in response to the patient's actual inhalation rate, and / or be pulsed and / or provide feedback to the patient.

### Brief Description of the Figures

Figure 1 shows the pneumatic components of a two pump device according to the invention.
Figure 2 shows the pneumatic components of an alternative embodiment of a two pump device.
Figure 3 shows a device according to the invention with a base unit and a handset.
Figure 4 shows the pneumatic components of a single pump device.

### Detailed Description of the Invention

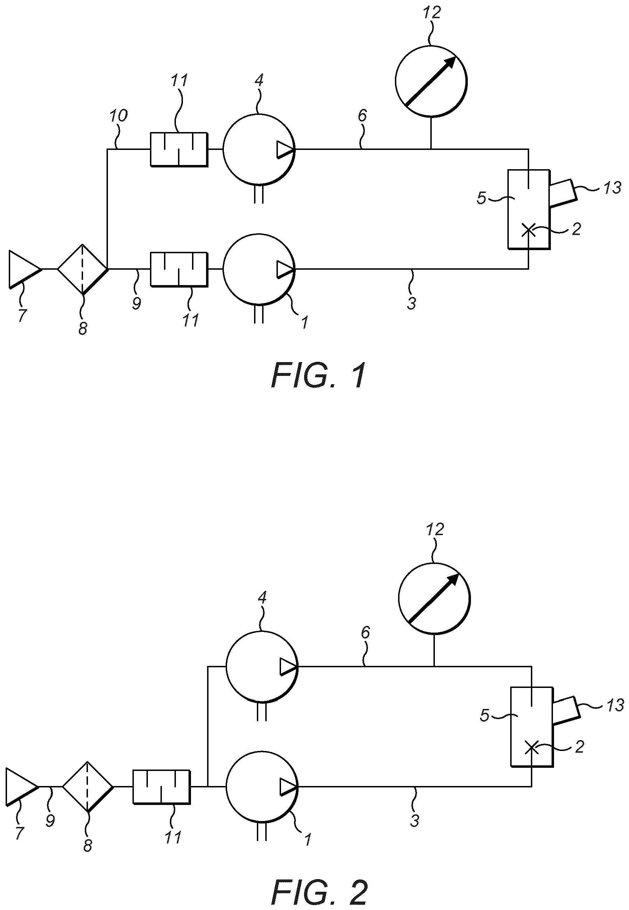

Figure 1 shows schematically the pneumatic components of an inhalation device according to the invention that provides an aerosol flow and a support flow. The device has a first pump **1** for providing the nebulizing air flow to a nebulization device **2,** located inside a mixing chamber **5.** The nebulization device may be a nebulizing nozzle connected to a reservoir that contains the liquid to be nebulized. The nebulizing nozzle generates an aerosol flow from the nebulizer air flow and the liquid. The first pump **1** is connected to the nebulization device **2** by a first air channel **3.** The device also has a a second pump **4** for providing the support flow. The device has a single air inlet **7,** with an air filter **8,** which feeds into separate air inlet channels **9, 10** for each of the pumps. Each pump has a muffler **11** which dampens oscillations in the air flow. The second pump **4** is connected to the mixing chamber **5** by a second air channel **6.** A pressure sensor **12** is also connected to the second air channel **6.** The aerosol flow is mixed with the support flow in the mixing chamber **5** to provide the total flow via a mouthpiece **13,** on which the patient inhales. The mouthpiece is unvented so that the device provides a closed pressure system in which the exact flow rate and volume received by the patient can be controlled. This contrasts with most nebulizers that have vented mouthpieces, and so are not closed systems.

In an alternative embodiment, shown in Figure 2, the pumps do not have separate mufflers. Instead, there is a single muffler **11.**

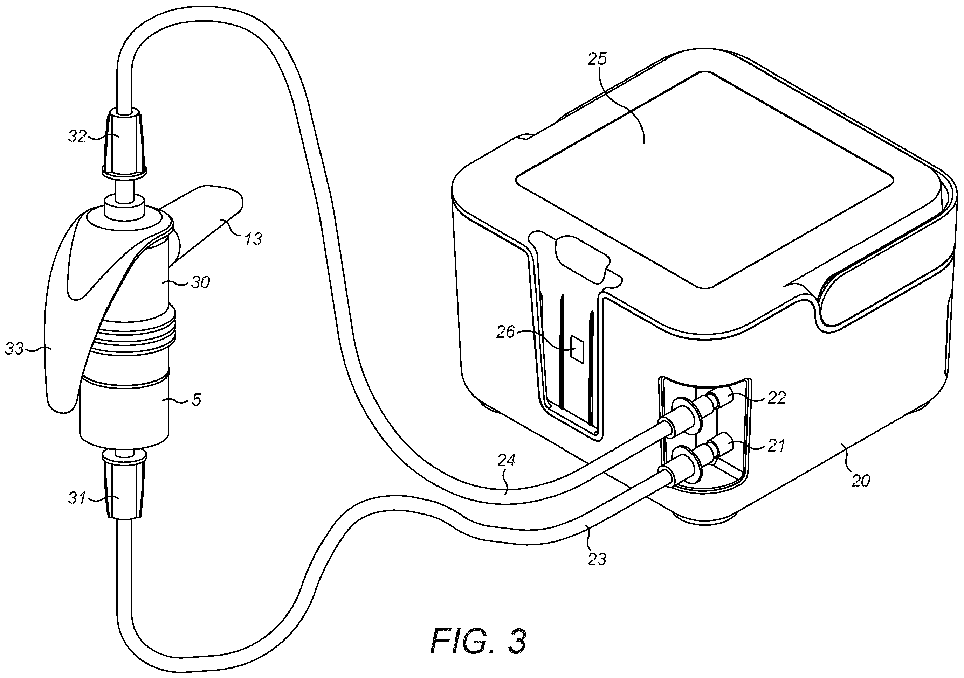

Figure 3 shows a device according to the invention. The device has a base unit **20** that contains the pumps, filter, muffler(s) and pressure sensor, and a handset **30** that contains an integral nebulizing nozzle and mixing chamber **5,** as well as a mouthpiece **13.** However, the nebulizing nozzle and the mixing chamber could also be separate components that are connected to each other via an air channel. The base unit has first and second air outlets in the form of tube connectors, **21, 22.** The handset **30** has first and second air inlets **31, 32.** Two flexible tubes **23, 24** connect the first and second air outlets **21, 22** of the base unit to the first and second air inlets **31, 32** of the handset. The tubes (together with pipes within the base unit) provide the air channels from the nebulizer pump and support flow pump respectively to the mixing chamber **5.** The handset **30** has a handle **33** for the patient to hold during use. The base unit **20** has a display screen **25** for providing instructions to the patient or care-giver and / or for providing feedback to guide the patient to inhale at the optimal rate.

The base unit **20** also has a treatment card reader **26** and an electronic controller (not visible in Figure 3). The treatment card is an RFID card which provides treatment parameters, such as the output flow rates from the pumps, length of the aerosol bolus etc.. The card reader reads the parameters from the treatment card using Near Field Communication (NFC), inputs them to the electronic controller. The controller also receives the output from the pressure sensor. The controller controls the pumps and the display screen.

The device provides an aerosol flow and a support flow, which can be independently varied by controlling the output flow rate from the first and second pumps. In particular, the aerosol flow can be turned on and off to provide an aerosol bolus. The aerosol bolus may be provided in a number of different ways. For example, the aerosol may be provided in the first part of the inhalation, after which the nebulizer pump is switched off, and the support flow is increased to maintain an approximately constant total flow for a second part of the inhalation (the flow is not exactly constant because of the ramp up / down times for the pumps). This provides a flow of aerosol-free air at the end of the inhalation, which ensures that all of the aerosol enters the lower parts of the patient's airway. Alternatively, there could be a first period of aerosol-free air, followed by the aerosol at the end of the inhalation, so that the aerosol is delivered to the upper parts of the airway. Another possibility is for there to be periods of aerosol-free air at the start and end of the breath with aerosol provided in the middle of the breath, so that it is delivered to the central parts of the airway. In each case, a constant total flow rate can be maintained by increasing the support flow at the times when the aerosol flow is stopped during inhalation, to compensate for the absence of the aerosol flow. This can be achieved by running the support flow pump at low power during the aerosol bolus and at higher power during the rest of the inhalation.

The device therefore provides the same functionality as the device of US 8 181 644, but without requiring the valves and Venturi nozzle. A limitation of the Venturi system is that the flow rates are fixed by the pressure required to operate the nebulizing nozzle and the geometry of the Venturi. In contrast, the separate support flow pump can be controlled to provide different flow rates. This allows the device to provide flow rates adapted to achieve a high deposition in the lung or peripheral airways and low deposition in the throat for specific patient populations; for example, 4-6 L/min for children from 2-8 years, 6-10 L/min for children from 8-12 years and 10-15 L/min for older children and adults. Thus, unlike conventional nebulizers, a single device can be used for patient groups with different airway capacities, for example due to their age or lung function.

The device is breath-actuated so that aerosol generation only takes place during inhalation. This avoids wasting the drug when the patient is exhaling or otherwise not at the mouthpiece. The pressure sensor measures the drop in pressure at the mouthpiece when the patient begins to inhale. The controller then switches on the nebulizer pump and / or the support flow pump (according to the way in which the aerosol bolus is to be delivered). The controller switches off the nebulizer pump and / or the support flow pump after a pre-set period of time. This time may be chosen to match the patient's natural inhalation time, and may be adjustable by the patient. The inhalation time for each breath has an effect on the total time of each treatment. For short inhalation times, the aerosol-free air part of the inhalation takes up a greater proportion of the inhalation time, so that the aerosol is delivered for a smaller proportion of the total inhalation time.

The device of the invention differs from ventilators and CPAP (continuous positive airway pressure) machines in which a positive pressure is continuously applied to the patient's upper airway in order to support the patient's breathing, thereby increasing the oxygen saturation in the blood and / or for preventing collapse of the upper airway (which occurs for example in patients with obstructive sleep apnea). In the device of the invention, the positive pressure is applied intermittently (i.e. as the patient inhales) for the purpose of guiding the patient to inhale with the correct breathing pattern (typically at a slow flow rate and for a long inhalation time) in order to maximize aerosol particle deposition in the lungs and / or peripheral airways and to minimize deposition in the throat.

The two-pump device has a number of further advantages. Firstly, the wear on the nebulizer pump and its energy consumption are reduced, since it only operates when actually providing the nebulizing gas flow. Secondly, the support flow pump acts as a valve which prevents exhalation back into the device (which could otherwise cause contamination), whereas in the device of US 8 181 644, an additional valve is needed for this purpose. Thirdly, it is straightforward to check whether both of the air flow channels **3, 6** are connected to the mixing chamber **5.** This is done by running the nebulizer pump **1** at a low voltage (in order to minimise the amount of drug that is nebulized) and checking whether the pressure sensor **12** records a drop in pressure. If either channel is not connected, then there is no pressure drop and the device can then display an error message to the patient or care-giver. Moreover, since the nebulizer pump and support flow pump are run independently, it is possible to automatically detect whether the tubes from the base unit to the handset are blocked (e.g. twisted) by monitoring the current consumption of each pump.

The two pumps could be of the same type, provided that they are capable of providing suitable flow rates (typically 5 - 7.5 L/min per pump, when both pumps are operating and 10 - 15 L/min when only the support flow pump is operating) against the different resistances of the nebulizer line (typically 1-2 bar, required to operate the nebulizing nozzle) and the support flow line (typically 0.1 - 0.2 bar). The nebulizer pump needs to provide a suitably high pressure for jet nebulization, but it does not need to provide the whole volumetric flow. For example, the nebulizer pump and support flow pumps can both be standard piston-type compressors, which could be of different sizes.

However, a major advantage of having a second pump (as opposed to a single pump with valves and a Venturi as in US 8 181 644) is that the nebulizer pump and the support flow pump can be different types of pump, and therefore can be chosen according to their particular function. Thus the second pump can be selected with characteristics that are best suited for providing the support flow, rather than the nebulizer flow. Also, separate mufflers can be used for each pump (as in the embodiment of Figure 1), in order to provide customized noise reduction, e.g. for different frequencies. It is important that the device is not noisy, since patients may use it frequently and / or for long treatment times.

In a preferred embodiment, the nebulizer pump is a piston-type compressor and the support flow pump is a multiple head diaphragm pump, such as a rolling diaphragm pump, a rotary vane pump or a scroll pump. As well as being capable of providing suitably high flow rates (e.g. 15 L/min), these types of pump produce a smoother flow (i.e. smaller pressure variations as a function of time) and are less noisy than a piston-type compressor, which has quite a "spiky" output. A smooth support flow is more comfortable for the patient.

Multiple head diaphragm pumps have a number of heads which operate in turn to provide the flow. For a given output flow, the pressure peaks caused by each head are smaller in amplitude and shorter in duration than for a pump with a single head, so the output is smoother. Rotary vane pumps also provide a smooth output, because the mulitple vanes similarly produce a series of small pressure peaks (the gaps between the vanes effectively provide multiple heads). However, rotary vane pumps require secondary filtering due to the risk of particulate generation from the vane tips. Scroll pumps also provide a smooth output with low noise, but can be quite heavy and expensive.

Using a support flow pump that produces a smooth output has the further advantage that it is not necessary to provide additional smoothing (as is done in the device of US 8 181 644 by the Venturi nozzle). The inherent resistance and compliance provided by the tubing that connects the support flow pump to the mouthpiece can be sufficient to damp the pressure oscillations. Thus the tubing that connects the base unit to the handset is chosen to be sufficiently large and compliant to damp the pressure oscillations and long enough for convenient handling by the patient or care-giver, whilst having a small enough internal volume to ensure rapid onset of pressure at the nebulizing nozzle when the nebulizer pump is switched on and rapid decay when switched off (e.g. within ~200 ms). Although the ramp up time is less important for the support flow pump, it is convenient that the tubing for both lines have similar lengths. The length of the tubing from the base unit to the handset is suitably from 1000 mm to 1300mm, for example about 1200mm. The inner diameter of the tubing is suitably about 3 to 5 mm, for example 4 mm.

A rolling diaphragm pump is the most preferred type of pump for the support flow because it is quiet, and provides a smooth output at relatively high flow rates and low pressures, whilst not having the disadvantages of rotary vane pumps and scroll pumps mentioned above. Rolling diaphragm pumps are also not prone to stalling when run at reduced speed, such as during the aerosol bolus. An example of a suitable rolling diaphragm pump is the Oken Seiko RFP55. A diaphragm pump is not suitable for the nebulizer flow however, because of the higher pressure required for nebulization.

The combination of a piston-type compressor as the nebulizer pump and a rolling diaphragm pump for the support flow pump is particularly preferred.

The pumps, and hence the total flow rate and the inhalation volume, are controlled by the electronic controller. Typically, the flow rate and the timing of the aerosol bolus is read from the treatment card, whereas the inhalation time, and hence the total volume, is input by the patient. Having two pumps provides additional ability to control the flow, by changing the support pump flow rate independently of the nebulizer pump flow rate, so that it can have a higher or lower flow rate. Thus, the support flow can be modulated by adjusting the flow from the support flow pump, without affecting the rate of nebulization (which is not possible in the device of US 8 181 644). For example, the support flow rate could be increased and decreased to mimic the patient's natural breathing pattern or in response to the patient's inhalation rate, or it could be pulsed, for example to help mobilize mucus in the patient's lungs and / or to open airways that are closed, e.g. filled with mucus.

Modulation of the support flow could also be used to give feedback to the patient. For example, the patient's inhalation effort can be monitored by means of the pressure sensor. If the patient is not inhaling quickly enough (i.e. with too little breathing effort) the flow rate could be increased slightly. This increase would be felt by the patient and would prompt them to inhale faster. Similarly, if the patient is inhaling too quickly, the flow rate could be reduced slightly. The patient would feel the drop in flow rate which would prompt them to inhale more slowly. Another possibility would be to pulse the flow to indicate that the target inhalation time or volume has nearly been reached. This allows the device to provide feedback to the patient in a simple manner, without requiring a screen which displays commands or shows the inhalation rate. Alternatively, this feedback could supplement visual feedback from the display screen to improve usability and convenience for the patient. Visual inhalation effort feedback can suitably be provided by a scale which indicates the target pressure and an acceptable pressure tolerance window on either side of the target pressure. The actual pressure measured by the system is then displayed to the patient and / or care-giver by means of an indicator (e.g. a dot) which moves up or down the scale as the actual pressure increases or decreases. The dot may change colour (e.g. become green) when the pressure is within the tolerance window and / or its brightness may increase the closer the actual pressure is to the target pressure.

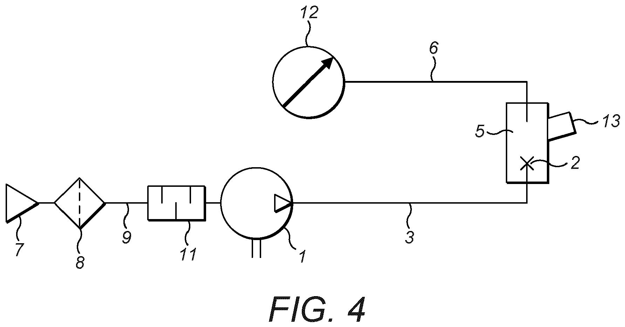

The device can easily be adapted specifically for lower flow rates, which are typical of pediatric patients, by removing the support flow pump and the associated part of the second channel upstream of the pressure sensor. As shown schematically in Figure 4, there is thus only a single pump **1** (typically a piston-type compressor) which supplies the nebulizing nozzle **2** and also provides the complete inhalation flow, so that there is no support flow. In contrast, the device of US 8 181 644 cannot easily be configured to run the nebulizer line alone. The nebulizer pump **1** can provide flow rates suitable for children (e.g. 6 L/min). The rest of the device architecture (nebulization nozzle **2,** air channel **3,** mixing chamber **5,** air inlet **7,** air filter **8,** air inlet channel **9,** muffler **11,** pressure sensor **12,** mouthpiece **13)** is essentially unchanged. The part of the second air channel **6** connecting the pressure sensor **12** to the mixing chamber **5** is retained in order to allow the pressure at the mouthpiece to be monitored for breath-actuation and / or providing feedback. The pump is preferably mounted at the centre of the base unit to provide an even weight distribution.

A common base design which can be easily configured for use with one or two pumps provides increased functionality without the need to create a separate device, which would be costly. This version of the device cannot provide the aerosol as a bolus that is preceded and / or followed by aerosol-free air since it only has one pump. However, this is not a significant drawback since delivering the aerosol as a bolus is less desirable in a pediatric device. Children have a much smaller total lung volume and time per inhalation than adults. Thus, for a fixed amount of aerosol to be delivered, the addition of a period of aerosol-free air would increase the overall treatment time substantially, which would not be acceptable for many pediatric treatments.

## Claims

1. A device for providing a flow of aerosol and / or air, comprising
• a base unit (20) containing
o a first pump (1) for providing a nebulizer air flow,
o a first channel (3) that connects the first pump (1) to a first air outlet (21),
o a second pump (4) for providing a support air flow, which is a multiple head diaphragm pump, such as a rolling diaphragm pump; or a rotary vane pump; or a scroll pump,
o a second channel (6) that connects the second pump (2) to a second air outlet (22),
o a pressure sensor (12) connected to the second channel (6), and
o a controller for controlling the first and second pumps;
• a handset (30) containing
o a reservoir for liquid to be nebulized,
o a mouthpiece (13), and
o a mixing chamber (5) having a first air inlet (31) connected to a nebulizing nozzle (2) for generating an aerosol flow from the nebulizer air flow and the liquid, and a second air inlet (32);
• a first tube (23) connecting the first air outlet (21) to the first air inlet (31); and
• a second tube (24) connecting the second air outlet (22) to the second air inlet (32).

2. A device according to claim 1, wherein the first pump (1) is a piston-type compressor.

3. A device according to claim 1 or claim 2, wherein the second pump (4) is a rolling diaphragm pump.

4. A device according to any of claims 1 to 3 further comprising a display screen (25) for providing instructions and / or feedback.

5. A device according to any of claims 1 to 4 wherein the first and / or second tubes (23, 24) are from 1000 mm to 1300 mm in length and have an inner diameter of from 3 to 5 mm.

6. A device according to any of claims 1 to 5 which is configured to provide, during an inhalation by the patient, an aerosol period in which there is both support flow and aerosol flow so that aerosol is delivered to the patient, and one or more aerosol-free periods when there is support flow but no aerosol flow, so that only air is delivered to the patient.

7. A device according to claim 6, which is configured to provide, in each inhalation:
• an aerosol period followed by an aerosol-free period; or
• an aerosol-free period followed by an aerosol period; or
• a first aerosol-free period, followed by an aerosol period, followed by a second aerosol-free period.

8. A device according to claim 6 or claim 7 which is configured to increase the support flow rate during the aerosol-free period(s) so that the total flow rate is approximately constant throughout the inhalation.

9. A device according to any of claims 6 to 8 which is configured to vary the support flow rate so that the total flow rate mimics the patient's natural breathing pattern.

10. A device according to any of claims 6 to 8 which is configured to vary the support flow rate in response to the patient's inhalation rate.

11. A device according to any of claims 6 to 8 which is configured to pulse the support flow.

12. A device according to any of claims 6 to 8 which is configured to provide feedback to the patient through the support flow.

## Patentansprüche

1. Vorrichtung zum Bereitstellen eines Stroms von Aerosol und/oder Luft, umfassend
• eine Basiseinheit (20), die enthält
o eine erste Pumpe (1) zum Bereitstellen eines Verneblerluftstroms,
o einen ersten Kanal (3), der die erste Pumpe (1) mit einem ersten Luftauslass (21) verbindet,
o eine zweite Pumpe (4) zum Bereitstellen eines Unterstützungsluftstroms, die eine Membranpumpe mit mehreren Köpfen, wie eine Rollmembranpumpe, oder eine Drehschieberpumpe oder eine Scrollpumpe ist,
o einen zweiten Kanal (6), der die zweite Pumpe (2) mit einem zweiten Luftauslass (22) verbindet,
o einen Drucksensor (12), der mit dem zweiten Kanal (6) verbunden ist, und
o eine Steuerung zum Steuern der ersten und der zweiten Pumpe;
• ein Handgerät (30), das enthält
o einen Behälter für die zu vernebelnde Flüssigkeit,
o ein Mundstück (13), und
o eine Mischkammer (5) mit einem ersten Lufteinlass (31), der mit einer Verneblungsdüse (2) zum Erzeugen eines Aerosolstroms aus dem Verneblerluftstrom und der Flüssigkeit verbunden ist, und einem zweiten Lufteinlass (32);
• einen ersten Schlauch (23), der den ersten Luftauslass (21) mit dem ersten Lufteinlass (31) verbindet; und
• einen zweiten Schlauch (24), der den zweiten Luftauslass (22) mit dem zweiten Lufteinlass (32) verbindet.

2. Vorrichtung nach Anspruch 1, wobei die erste Pumpe (1) ein Kolbenverdichter ist.

3. Vorrichtung nach Anspruch 1 oder Anspruch 2, wobei die zweite Pumpe (4) eine Rollmembranpumpe ist.

4. Vorrichtung nach einem der Ansprüche 1 bis 3, ferner umfassend einen Anzeigebildschirm (25) zum Bereitstellen von Anweisungen und/oder Rückmeldungen.

5. Vorrichtung nach einem der Ansprüche 1 bis 4, wobei der erste und/oder der zweite Schlauch (23, 24) von 1000 mm bis 1300 mm lang ist und einen Innendurchmesser von 3 bis 5 mm hat.

6. Vorrichtung nach einem der Ansprüche 1 bis 5, die dazu ausgelegt ist, während einer Inhalation durch den Patienten eine Aerosolperiode bereitzustellen, in der es sowohl einen Unterstützungsstrom als auch einen Aerosolstrom gibt, so dass das Aerosol an den Patienten geliefert wird, und einen oder mehrere aerosolfreie Perioden, in denen es einen Unterstützungsstrom, aber keinen Aerosolstrom gibt, so dass nur Luft an den Patienten geliefert wird.

7. Vorrichtung nach Anspruch 6, die dazu ausgelegt ist, bei jeder Inhalation bereitzustellen:
• eine Aerosolperiode, gefolgt von einer aerosolfreien Periode; oder
• eine aerosolfreie Periode, gefolgt von einer Aerosolperiode; oder
• eine erste aerosolfreie Periode, gefolgt von einer Aerosolperiode, gefolgt von einer zweiten aerosolfreien Periode.

8. Vorrichtung nach Anspruch 6 oder Anspruch 7, die dazu ausgelegt ist, die Unterstützungsstromrate während der aerosolfreien Periode(n) zu erhöhen, so dass die Gesamtstromrate während der gesamten Inhalation annähernd konstant ist.

9. Vorrichtung nach einem der Ansprüche 6 bis 8, die dazu ausgelegt ist, die Unterstützungsstromrate zu variieren, so dass die Gesamtstromrate das natürliche Atmungsmuster des Patienten nachbildet.

10. Vorrichtung nach einem der Ansprüche 6 bis 8, die dazu ausgelegt ist, die Unterstützungsstromrate in Reaktion auf die Inhalationsrate des Patienten zu variieren.

11. Vorrichtung nach einem der Ansprüche 6 bis 8, die dazu ausgelegt ist, den Unterstützungsstrom zu pulsieren.

12. Vorrichtung nach einem der Ansprüche 6 bis 8, die dazu ausgelegt ist, dem Patienten über den Unterstützungsstrom Rückmeldung bereitzustellen.

## Revendications

1. Dispositif permettant de fournir un flux d'aérosol et/ou d'air, comprenant
• une unité de base (20) contenant
∘ une première pompe (1) permettant de fournir un flux d'air de nébuliseur,
∘ un premier canal (3) qui relie la première pompe (1) à une première sortie d'air (21),
∘ une seconde pompe (4) permettant de fournir un flux d'air de support, qui est une pompe à membrane à têtes multiples, telle qu'une pompe à membrane déroulante ; ou une pompe à palettes ; ou une pompe à spirale,
∘ un second canal (6) qui relie la seconde pompe (2) à une seconde sortie d'air (22),
∘ un capteur de pression (12) relié au second canal (6), et
∘ un dispositif de commande pour commander les première et seconde pompes ;
• une télécommande manuelle (30) contenant
∘ un réservoir pour le liquide à nébuliser,
∘ un embout buccal (13), et
∘ une chambre de mélange (5) comportant une première entrée d'air (31) reliée à une buse de nébulisation (2) pour générer un flux d'aérosol à partir du flux d'air de nébuliseur et du liquide, et une seconde entrée d'air (32) ;
• un premier tube (23) reliant la première sortie d'air (21) à la première entrée d'air (31) ; et
• un second tube (24) reliant la seconde sortie d'air (22) à la seconde entrée d'air (32).

2. Dispositif selon la revendication 1, dans lequel la première pompe (1) est un compresseur de type à piston.

3. Dispositif selon la revendication 1 ou la revendication 2, dans lequel la seconde pompe (4) est une pompe à membrane déroulante.

4. Dispositif selon l'une quelconque des revendications 1 à 3, comprenant en outre un écran d'affichage (25) permettant de fournir des instructions et/ou une rétroaction.

5. Dispositif selon l'une quelconque des revendications 1 à 4, dans lequel les premier et/ou second tubes (23, 24) ont une longueur de 1 000 mm à 1 300 mm et ont un diamètre interne de 3 à 5 mm.

6. Dispositif selon l'une quelconque des revendications 1 à 5 qui est configuré pour fournir, pendant une inhalation par le patient, une période d'aérosol dans laquelle il y a à la fois un flux de support et un flux d'aérosol de sorte que l'aérosol soit administré au patient, et une ou plusieurs périodes exemptes d'aérosol lorsqu'il y a un flux de support mais pas de flux d'aérosol, de sorte que seul de l'air soit administré au patient.

7. Dispositif selon la revendication 6, qui est configuré pour fournir, dans chaque inhalation :
• une période avec aérosol suivie d'une période sans aérosol ; ou
• une période sans aérosol suivie d'une période avec aérosol ; ou
• une première période sans aérosol, suivie d'une période avec aérosol, suivie d'une seconde période sans aérosol.

8. Dispositif selon la revendication 6 ou la revendication 7 qui est configuré pour augmenter le débit de support pendant la ou les périodes sans aérosol de sorte que le débit total soit approximativement constant tout au long de l'inhalation.

9. Dispositif selon l'une quelconque des revendications 6 à 8 qui est configuré pour faire varier le débit de support de sorte que le débit total reproduise le schéma respiratoire naturel du patient.

10. Dispositif selon l'une quelconque des revendications 6 à 8 qui est configuré pour faire varier le débit de support en réponse à la fréquence d'inhalation du patient.

11. Dispositif selon l'une quelconque des revendications 6 à 8 qui est configuré pour donner des impulsions au débit de support.

12. Dispositif selon l'une quelconque des revendications 6 à 8 qui est configuré pour fournir une rétroaction au patient par l'intermédiaire du débit de support.
